# EUROPEAN PATENT APPLICATION

(11) **EP 4 799 605 A1**
(43) Date of publication of application: **02.09.2026**
(21) Application number: 24880928.7
(22) Date of filing: 20.03.2024
(51) Int. Cl.: A61K 8/9794, A61K 8/9789, A61K 8/99, A61K 8/02, A61Q 19/00, A61Q 19/02, A61Q 19/08

(54) **METHOD FOR PREPARING BIO-CELLULOSE MASK CLOTH AND BIO-CELLULOSE MASK CLOTH OBTAINED THEREBY**

(30) Priority: 27.10.2023 CN 202311424411
(71) Applicant: Yangshengtang (ANJI) Cosmetics Co., Ltd., Huzhou, Zhejiang 313399 (CN)
(72) Inventor: ZHAO, Shizhi, Huzhou, Zhejiang 313399 (CN); HUANG, Fengfei, Huzhou, Zhejiang 313399 (CN); ZHOU, Yue, Huzhou, Zhejiang 313399 (CN); HUANG, Jian, Huzhou, Zhejiang 313399 (CN); BAI, Xiaoyun, Huzhou, Zhejiang 313399 (CN)
(74) Representative: J A Kemp LLP
(86) International application number: PCT/CN2024/082558
(87) International publication number: WO 2025/086542

(57) **Abstract**

The invention provides a method for preparing a bio-fiber facial mask cloth, which comprises the following steps: (1) activating and passaging Acetobacter xylinum to obtain passaged Acetobacter xylinum culture liquid; (2) uniformly inoculating about 1-15%, preferably 5-10% of the passaged Acetobacter xylinum culture liquid into the culture medium, and fermenting at a temperature of about 25-35°C, preferably 25-30°C, for about 2-5 days, preferably 2-3 days, wherein the culture medium comprises about 18% or more, preferably about 27% or more, more preferably about 36% or more, and most preferably about 45% or more of birch sap and about 0-71%, preferably about 10-62% or more preferably about 30-53% of coconut water, wherein all the above content percentages are based on the total weight of the fermentation broth; and (3) collecting the membrane.

## Description

### Technical field

The invention relates to a method for preparing a bio-fiber facial mask cloth and a bio-fiber facial mask cloth obtained therefrom.

### Background art

At present, the beauty and skin care facial mask on the market is usually made of man-made fiber non-woven fabric or chemical synthetic fiber non-woven fabric via subsequent processing. However, these non-woven facial masks have some defects and shortcomings, such as poor affinity with the skin, general permeability, poor penetration effect of active substances, fibers arranging in a certain direction so they are easy to split from a right angle, etc; and when the facial mask is left on for a long time, the phenomenon of "reverse suction" will appear. In addition, non-woven fabric as mask cloth can only be used as the carrier for mask solution, without other additional value, and it is derived from petrochemical industry, thereby is not environment friendly.

Bio-fiber facial mask is a new type of facial mask, which is a bio-cellulose membrane made by natural fermentation of microorganisms, has a skin like function, and can penetrate oxygen and isolate bacteria. Coconut water is the earliest and currently widely used raw material for producing this type of bio-cellulose membrane. In recent years, bio-cellulose has been widely used in many fields such as healthcare, textiles, environmental protection, and papermaking due to its excellent properties, resulting in a huge demand for it. However, coconut water, as the main raw material for its production, is geographically limited, therefore, finding an alternative to coconut water as a raw material for producing bio-cellulose has become a research hotspot.

Chinese patent application No. 201110176434.3 discloses a method for preparing bi-ocellulose using inulin as a carbon source, which has the advantages of wide raw material sources, low cost, and strong operability. The yield of using inulin to produce bio-cellulose is higher than that of using other glucose and/or fructose, and can reach up to twice the yield. However, the organic fiber membrane obtained from inulin has the lowest tensile strength and moisture content.

Chinese patent application No. 201510942260.5 discloses a method for preparing bio-cellulose using optimized watermelon juice culture medium. By screening the main components in the watermelon juice culture medium, determining key factors, and optimizing, the obtained bio-cellulose has better water-holding capacity, crystallinity, and thermal stability than the basic watermelon juice culture medium.

Chinese patent application No. 201310314954.5 discloses a method for preparing a bio-fiber facial mask with anti-radiation effect, in which the total flavonoids are extracted from natural plants by ultrasonic method, and the alcohol solution containing the total flavonoids is used as the culture medium to ferment bio-cellulose materials to prepare a bio-fiber facial mask, which can eliminate excessive reactive oxygen free radicals on the face and has anti-radiation efficacy.

However, the bio-cellulose membrane obtained by fermentation in the existing technologies can only improve one or two basic properties of fibers, such as crystallinity and tensile strength, and this improvement is not significant. Moreover, the existing bio-cellulose membrane cannot meet the requirements of consumers for mild and non-irritating mask cloth, as well as improving skincare efficacy.

Therefore, there is a need of a new bio-fiber facial mask cloth that can overcome the shortcomings of the bio-fiber facial mask cloth in the prior art and its preparation method.

### Brief description of the drawings

Figs. 1 and 2 respectively show the situation of sample 1 according to the present invention and comparative samples 2 and 3 for promoting the penetration of active substance myopeptide.
Fig. 3 shows the efficacy of sample 1 according to the present invention and comparative sample 3 in improving skin firmness.

### Summary of the invention

In one aspect, the invention provides a method for preparing a bio-fiber facial mask cloth, which comprises the following steps:
(1) activating and passaging Acetobacter xylinum to obtain passaged Acetobacter xylinum culture liquid;
(2) uniformly inoculating about 1-15%, preferably 5-10% of the passaged Acetobacter xylinum culture liquid into a culture medium, and fermenting at a temperature of about 25-35°C, preferably 25-30°C, for about 2-5 days, preferably 2-3 days, wherein the culture medium contains about 18% or more, preferably about 27% or more, more preferably about 36% or more, and most preferably about 45% or more of birch sap and about 0-71%, preferably about 10-62%, more preferably about 30-53% of coconut water, wherein all the above amount percentages are based on the total weight of the fermentation broth (the fermentation broth consists of the culture medium and the Acetobacter xylinum culture liquid); and
(3) collecting the obtained membrane sheet (i.e. mask sheet).

The activation and passage of Acetobacter xylinum in step (1) are known in this field. For example, they are usually done as follows:

### a. activation

An appropriate amount of Acetobacter xylinum is taken from a refrigerator, thawed at room temperature, and added to a first liquid culture medium, then, to the culture medium was added about 5-15ml of ethanol having a concentration of 95% by volume, and cultured in a shaker at about 25-35°C and 170-190rpm for about 36-54 hours, to obtain activated Acetobacter xylinum culture liquid;

### b. strain passage

Approximately 5-15% of the activated Acetobacter xylinum culture liquid, based on the total weight of the culture medium, is added to the first liquid culture medium, then, to the culture medium was added about 5-15ml of ethanol having a concentration of 95% by volume, and cultured in a shaker at about 25-35°C and 170-190rpm for about 18-30 hours, to obtain the passaged Acetobacter xylinum culture liquid;

The Acetobacter xylinum is a commonly used strain in the field of producing bio-fiber facial mask cloth by fermentation, with properties known in the field, and are commercially available, for example, available from LMAI Bio, Shanghai.

The first liquid culture medium is known in the art and typically contains about 2-10% carbon source, about 0.1-10% nitrogen source, and about 0-10% inorganic salt by weight, and balance being pure water. The carbon source is known in the art, including, for example, glucose, lactose, sucrose, ethanol, hexanediol, glycerol lactose, and mannose etc. The nitrogen source is known in the art, including, for example, yeast extract, peptone, L-glutamate, L-aspartate, L-alanine etc. The inorganic salts are known in the art, including, for example, sodium pyrophosphate, sodium chloride, potassium chloride, magnesium sulfate etc. Usually, a buffer solution such as phosphate buffer can be used to adjust the pH value of the first liquid culture medium to, e.g. about 4.0-6.0. Typically, the first liquid culture medium can be sterilized in high-pressure steam at about 121°C for about 30 mins.

The culture medium used in the step (2) contains about 18% or more, preferably about 27% or more, more preferably about 36% or more, and most preferably about 45% or more of birch sap, and about 0-71%, preferably about 10-62%, more preferably 30-53% of coconut water, based on the total weight of the fermentation broth.

The birch sap is obtained from Betula Betulaceae, including varieties of Betala alba, Betula pubescens, Betula Pendula, and Betula platyphylla etc. The birch sap is a colorless, transparent and nutritious sap without precipitates or impurities obtained by artificially drilling and collecting from the base of the trunk of a birch tree from the time when the snow begins to melt to the time when the trees foliate. The birch sap is commercially available and used as it is, for example, it can be purchased from Daxinganling Chaoyue Wild Berry Development Co., Ltd.

The birch sap that can be used in the present invention is birch sap stock solution, fermented birch sap, or concentrated birch sap. The concentration degree of the concentrated birch sap is about 1.05-10.0 times, preferably about 2.0-6.0 times. The fermented birch sap can be fermented birch sap known in the art, such as birch sap fermented by Inonotus Obliquus disclosed in Chinese patent CN110734932B, birch sap fermented by saccharomycetes disclosed in CN112842976A and CN112842953A, all of which are incorporated herein by reference.

The concentrated birch sap is obtained by concentrating the above-mentioned commercially available products. The concentration methods are known in the art, for example, heating concentration, low-temperature and vacuum concentration, and membrane concentration. In the present invention, the concentration is preferably performed by a low-temperature freeze concentration or membrane concentration process. For example, a commercially available birch sap stock solution is fed into a low-temperature drying device, cooled to -40 to -70°C, and vacuumed to 0.1-30Pa for low-temperature and vacuum concentration process, to obtain concentrated birch sap having different concentration degrees.

The coconut water is extracted from fresh coconuts. Coconut is mainly grown in the southeastern coastal areas of Hainan. Coconut water can be directly extracted from the coconut or commercially available and used as it is, for example, it can be purchased from Wenchang Yedao Industrial Co., Ltd.

As needed, birch sap and coconut water can be filtered through a filter cloth with a mesh size of about 300 or more to remove impurities; optionally, the culture medium is sterilized for about 10 minutes at a temperature of about ≥95°C.

The culture medium can contain, in addition to birch sap and coconut water, carbon source, inorganic salt, pH regulator etc., these components and amounts thereof are known in the art.

The carbon source includes, for example, glucose, white granulated sugar, fructose, sucrose, galactose, glycerol lactose, and mannose. The amount of the sugar source can be chosen by those skilled in the art, typically ranging from about 0.5-1.5%, preferably about 0.7-1%, based on the total weight of the fermentation broth.

The inorganic salt includes, for example, potassium dihydrogen phosphate, sodium dihydrogen phosphate, dipotassium hydrogen phosphate, disodium hydrogen phosphate, diammonium bicitrate, sodium acetate, manganese sulfate, magnesium sulfate, and potassium nitrate. The amount of the inorganic salt can be chosen by those skilled in the art, typically ranging from about 0-2%, preferably about 0.2-1%, based on the total weight of the fermentation broth.

The pH regulator includes, for example, lactic acid, citric acid, ammonia, sodium lactate, glacial acetic acid, sodium citrate, and sodium hydroxide, with glacial acetic acid being preferred. The pH regulator is added to the culture medium to adjust the pH of the culture medium to about 2.0-5.5, preferably about 3.0-4.5.

In the fermentation process of the above step (2), about 1-15%, preferably about 5-10% of the passaged Acetobacter xylinum culture liquid, based on the total weight of the fermentation broth, is uniformly inoculated into the culture medium and fermented at a temperature of about 25-35°C, preferably about 25-30°C, for about 2-5 days, preferably about 2-3 days, to obtain a bio-fiber membrane sheet.

The membrane sheet collection of the above step (3) is known in the art, and usually includes, for example, washing, spreading, squeezing out water, cutting and shaping etc. of the obtained bio-fiber membrane sheet, to obtain a bio-fiber facial mask cloth.

The bio-fiber facial mask cloth obtained by the preparation method of the invention usually has an average thickness of about 2.5-6.0mm, preferably 3.0-6.0mm, a mass of about 280-900g/L, preferably 550-900g/L (here, the mass refers to a wet weight, and the unit g/L refers to the mass of the bio-fiber facial mask produced per liter of fermentation broth), and a tensile strength of about 0.5-4.5N/mm², preferably 0.9-4.0N/mm².

Moreover, the bio-fiber facial mask cloth obtained by the preparation of the invention has excellent permeability promotion. For example, compared with the permeability of the bio-fiber facial mask cloth obtained by fermentation of coconut water only, the bio-fiber facial mask cloth of the invention can improve the skin permeability of carnosine as an active substance by more than 2.5 times; compared with the permeability of ordinary Tencel membrane cloth, the bio-fiber facial mask cloth of the invention can improve the skin permeability of carnosine as an active substance by more than 4 times.

In addition, the yield of the bio-fiber facial mask cloth prepared by the method of the invention can reach about 4g/L or more, wherein the unit g/L refers to the dry weight of the bio-fiber facial mask cloth produced per liter of fermentation broth. Compared with the prior art method of only using coconut water for fermentation to prepare bio-fiber facial mask cloth, the preparation method of the present invention significantly improves the yield of bio-fiber facial mask cloth, generally increasing the yield by about 200-270%. The best results are obtained when the fermentation broth contains about 40-50% of the birch sap stock solution or fermented birch sap.

In the other aspect, the invention provides a bio-fiber facial mask cloth obtained by the above method, which has the average thickness and quality as described above, as well as excellent tensile strength and permeability promotion.

The inside of the bio-fiber facial mask cloth is a unique dense three-dimensional network structure composed of cellulose and has many micropores. This unique structure in combination with the activity of fermented birch sap itself enables the bio-fiber facial mask cloth to obtain many beneficial properties when it contacts with the skin, including, for example, excellent conformity, moisture retention, softness, high water retention, good biocompatibility and biodegradability, mild and non-irritating, and excellent permeability promoting performance.

The bio-fiber facial mask cloth can be used to load the commonly used facial mask liquid known in the art.

At the same time, the bio-fiber facial mask cloth can not only be used as the carrier for facial mask liquid, but also has good cosmetic efficacy, and can calm, soothe and repair the skin, especially suitable for the skin repair after sunburn and medical beauty projects.

In addition, the bio-fiber facial mask cloth can also be used as a health food, which can regulate the body function, promote intestinal peristalsis, improve the skin state, whiten and delay skin aging.

### Examples

The present invention is further described in detail below with reference to examples. However, it should be understood that these examples and comparative examples are only used to specifically illustrate the present invention, and should not be understood to limit the scope of the appended claims of the present invention in any manner.

### Example 1: Preparation of bio-fiber facial mask cloth

In the example, birch sap or its mixture with coconut water, Acetobacter xylinum, and the formulation of the culture medium in Table 1 below (wherein the remaining components are 0.8% white granulated sugar and 0.2% sodium acetate, respectively) are used to prepare a bio-fiber facial mask cloth, in which the amount was based on the total weight of the fermentation broth (i.e., the total weight of the culture medium and the Acetobacter xylinum culture liquid).

**Table 1**

| Examples | Birch sap amount | Coconut water amount | Remaining components | Strain amount | pH |
|---|---|---|---|---|---|
| 1 | 89% | 0 | 1% | 10% | 3.84 |
| 2 | 45% | 44% | 1% | 10% | 3.49 |
| 3 | 27% | 62% | 1% | 10% | 3.47 |
| 4 | 18% | 71% | 1% | 10% | 3.45 |
| 5 (comparative) | 9% | 80% | 1% | 10% | 3.41 |
| 6 (comparative) | 0 | 89% | 1% | 10% | 3.37 |

The preparation steps were as follows:

### (1) activation and passage of Acetobacter xylinum

### a. Activation

10ml of the preserved strain Acetobacter xylinum was taken from a refrigerator and thawed at room temperature, added to 500g of the first liquid culture medium, then to the culture medium was added 10mL of ethanol having a concentration of 95% by volume, and activated and cultured in a shaker at 30°C and 180rpm for 50 hours to obtain activated Acetobacter xylinum culture liquid.

The first liquid culture medium comprises 10g of glucose, 5g of yeast extract, 2.5g of peptone, 2.5g of sodium pyrophosphate, and balance pure water, with a total amount of 500g of culture medium. The culture medium was adjusted with phosphate buffer to a pH of 4.5, placed in a 2L conical flask, and sealed with a cotton stopper, sterilized in high-pressure steam at 121°C for 30 minutes.

### b. Passage

20ml of the activated Acetobacter xylinum culture liquid was taken from the conical flask and added to 500g of the first liquid culture medium, and to the culture medium was added 10mL of ethanol having a concentration of 95% by volume, and cultured in a shaker at 30°C and 180rpm for 20 hours to obtain the passaged Acetobacter xylinum culture liquid.

### (2) Inoculation and fermentation culture

10% (based on the total weight of the fermentation broth) of the passaged Acetobacter xylinum culture liquid was evenly inoculated into the culture medium in a 32 x 24cm fermentation plate, and cultured for 3 days at a temperature of 30°C. The formulation of the culture medium was described in Table 1 above.

### (3) Membrane collection

The formed bio-fiber membrane sheet (mask sheet) was taken out, the excess culture medium was washed off, and the membrane sheet was soaked in a 0.5% by mass of sodium percarbonate solution and washed until there was no acetic acid odor, then the membrane sheet was taken out and the excess sodium percarbonate solution was washed off, after squeezing out water, the thickness, weight, and yield of the obtained bio-fiber membrane sheet (which size is the size of the fermentation plate 32x24cm) were measured, and the membrane sheet was further cut to obtain the desired bio-fiber facial mask sheet, wherein the obtained values were the average of six parallel experiments. The results were shown in Table 2 below.

**Table 2**

| Examples | Thickness (mm) | Weight(g) | Yield (g/L) |
|---|---|---|---|
| 1 | 4.70 | 772 | 3.9 |
| 2 | 5.07 | 865 | 4.1 |
| 3 | 3.31 | 630 | 3.2 |
| 4 | 3.15 | 587 | 3.0 |
| 5(comparative) | 2.35 | 342 | 1.8 |
| 6(comparative) | 2.04 | 283 | 1.5 |

### Example 2: Preparation of bio-fiber facial mask cloth

In the example, the birch sap fermented by Inonotus obliquus as disclosed in Chinese patent CN110734932B or its mixture with coconut water, and the same Acetobacter xylinum culture liquid as in Example 1, and the formulation of the culture medium in Table 3 below (wherein the remaining components were 0.8% of white granulated sugar and 0.2% of sodium acetate, respectively) were used to prepare the bio-fiber facial mask cloth, in which the amount was based on the total weight of the fermentation broth (i.e., the total weight of the culture medium and the Acetobacter xylinum culture liquid).

**Table 3**

| Examples | Fermented Birch sap amount | Coconut water amount | Remaining components | Strain amount | pH |
|---|---|---|---|---|---|
| 1 | 89% | 0% | 1% | 10% | 3.89 |
| 2 | 45% | 44% | 1% | 10% | 3.61 |
| 3 | 27% | 62% | 1% | 10% | 3.47 |
| 4 | 18% | 71% | 1% | 10% | 3.47 |
| 5(comparative) | 9% | 80% | 1% | 10% | 3.40 |
| 6(comparative) | 0% | 89% | 1% | 10% | 3.37 |

The preparation steps were as described in Example 1.

The thickness, weight and yield of the obtained bio-fiber facial mask sheet were shown in Table 4 below.

**Table 4**

| Examples | Thickness (mm) | Weight (g) | Yield (g/L) |
|---|---|---|---|
| 1 | 4.88 | 817 | 4.0 |
| 2 | 5.90 | 896 | 4.3 |
| 3 | 3.85 | 682 | 3.7 |
| 4 | 3.50 | 596 | 3.1 |
| 5(comparative) | 2.82 | 374 | 1.9 |
| 6(comparative) | 2.04 | 283 | 1.5 |

### Example 3: Tensile property of bio-fiber facial mask cloth

In the example, the tensile properties of the bio-fiber facial mask cloth prepared in Examples 1 and 2 were tested according to the standard GB/T 24218.3 "Test Method for Tensile Strength"). The results were shown in Table 5, wherein the tensile strength values werethe average of the results of six parallel experiments.

**Table 5**

| Examples in Example 1 | Tensile strength(N/mm²) | Examples in Example 2 | Tensile strength (N/mm²) |
|---|---|---|---|
| 1 | 1.216 | 1 | 4.020 |
| 2 | 1.052 | 2 | 3.642 |
| 3 | 0.980 | 3 | 2.295 |
| 4 | 0.927 | 4 | 3.275 |
| 5(comparative) | 0.734 | 5(comparative) | 1.809 |
| 6(comparative) | 0.741 | 6(comparative) | 0.741 |

From the results of the thickness, weight, tensile strength, and yield of the bio-fiber facial mask sheet obtained above, it can be seen that the best results were obtained when the fermentation broth contains 45% of birch sap stock solution or fermented birch sap. Particularly, the yield of dry bio-fiber facial mask obtained from the fermentation broth containing 45% fermented birch sap reached 4.3g/L (example 2 in Example 2), which is much better than the yield of 1.5g/L obtained from the fermentation broth containing only coconut water (example 6 in Example 2).

### Example 4: Test of efficacy of bio-fiber facial mask cloth

In the example, the following efficacy of the prepared bio-fiber facial mask cloth were further tested.

In the test, sample 1 was the bio-fiber facial mask sheet of example 2 in Example 1 (i.e., the bio-fiber facial mask sheet obtained by fermentation with the fermentation broth containing 45% birch sap), and sample 2 was the ordinary Tencel facial mask cloth (available from Shanghai Meanlove BIO-TECH Co., Ltd.), Sample 3 was the bio-fiber facial mask sheet of example 6 in Example 1 (i.e. the bio-fiber facial mask sheet obtained by fermentation with the fermentation broth containing only coconut juice but not birch sap).

In this test, the formulation of the facial mask liquid used was as follows:

**Table 6**

| No. | Raw material name | wt% |
|---|---|---|
| 1 | Deionized water | Balance to 100% |
| 2 | Glycerol | 3 |
| 3 | Butanediol | 3 |
| 4 | Allantoin | 0.1 |
| 5 | Xanthan gum | 0.05 |
| 6 | Carbomer | 0.12 |
| 7 | D-panthenol | 0.3 |
| 8 | Betaine | 2 |
| 9 | Sodium hyaluronate | 0.05 |
| 10 | Hexanediol | 0.5 |
| 11 | P-hydroxyacetophenone | 0.5 |
| 12 | Tromethamine | 0.08 |
| 13 | Carnosine | 0.2 |
| 14 | Oat kernel extract | 1 |

### 1. Test of penetration promoting efficacy

This test was conducted on three subjects. On the day of the test, the subjects washed the inner sides of both forearms with clean water and equilibrated in a constant temperature and humidity laboratory for 20 minutes, then, three test areas (area: 4 x 4cm²) were marked on the inner sides of both forearms of the subjects, the technicians applied the samples to the test areas and removed them after 30 minutes. The contents of cosmetic ingredients in the test area were tested before use of samples (initial value) and 1 hour after single use, respectively, wherein the instrument used was a skin ingredient analyzer (gen2-SCA, RiverD, Netherlands). Before testing, the test area was wiped three times with dust-free paper to remove any residue of the sample on the skin surface.

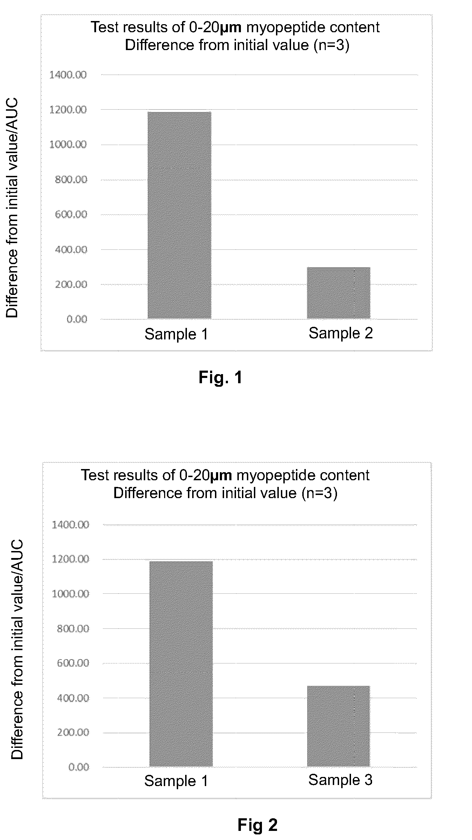

The results were shown in Figures 1 and 2. The results showed that after 1 hour of single use of the samples, the contents of active substance carnosine in the testing area 0-20µm caused by sample 1 was higher than thoese of samples 2 and 3. Among them, the permeability values of carnosine caused by sample 1 was 4.00 times that of sample 2 and 2.52 times that of sample 3.

### 2. Test of efficacy of improving skin firmness

In this test, based on a full face design, 33 eligible female volunteers participated. During the test, the volunteers can continue to use their own original skin care products and skin care habits, and use test samples instead of their original facial mask products. The test samples were used every other night for four consecutive weeks. Before using the test samples and after 4 weeks of use, skin sagging imaging tests were performed on the randomly selected mandibular area of the volunteers, using a skin rapid optical imaging system (AEVA HE, Breuckmann, Germany).

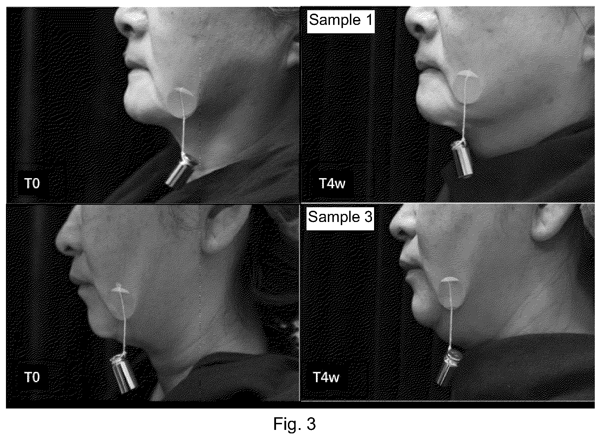

The results were shown in Figure 3. The results showed that under the use of same facial mask liquid, Sample 1 exhibited a significant improvement in skin tightness, while Sample 3 only exhibited slightly improvement.

The technical solutions of the above-mentioned examples were preferred embodiments of the present invention. Several improvements and changes can be made without departing from the principle of the present invention, and these improvements and changes should also be regarded as falling within the protection scope of the present invention.

## Claims

1. A method for preparing a bio-fiber facial mask cloth, which comprises the following steps:
(1) activating and passaging Acetobacter xylinum to obtain passaged Acetobacter xylinum culture liquid;
(2) uniformly inoculating 1-15% of the passaged Acetobacter xylinum culture liquid into a culture medium, and fermenting at a temperature of about 25-35°C for about 2-5 days, wherein the culture medium contains 18% or more of birch sap and 0-71% of coconut water, wherein all the above content percentages are based on the total weight of the fermentation broth; and
(3) collecting the membrane.

2. The method according to claim 1, wherein the culture medium comprises 27% or more of birch sap.

3. The method according to claim 2, wherein the culture medium comprises 36% or more of birch sap.

4. The method according to claim 3, wherein the culture medium comprises 45% or more of birch sap.

5. The method according to any one of claims 1-4, wherein the culture medium comprises 10-62% coconut water.

6. The method according to claim 5, wherein the culture medium comprises 30-53% coconut water.

7. The method according to any one of claims 1-6, wherein 5-10% of the passaged Acetobacter xylinum culture liquid is uniformly inoculated into the culture medium.

8. The method according to any one of claims 1-7, wherein the fermentation is carried out at a temperature of 25-30°C for 2-3 days.

9. The method according to any one of claims 1-8, wherein the birch sap is a birch sap stock solution, a fermented birch sap, or a concentrated birch sap with a concentration degree of 1.05-10.0 times.

10. The method according to claim 9, wherein the concentration degree of the concentrated birch sap is 2.0-6.0 times.

11. The method according to claim 9, wherein the fermented birch sap is either birch sap fermented by Inonotus obliquus or Saccharomycetes.

12. The method according to any one of claims 1-11, wherein the culture medium further comprises a carbon source, an inorganic salt, and/or pH regulator.

13. Abio-fiber facial mask cloth obtained by the method of any one of claims 1-12.
